**Europäisches Patentamt**

**European Patent Office**

**Office européen des brevets**

(19)

(11) Numéro de publication : **0 379 530 B1**

(12) **FASCICULE DE BREVET EUROPEEN**

(45) Date de publication du fascicule du brevet :
08.07.92 Bulletin 92/28

(51) Int. Cl.$^5$ : **A61F 5/00**

(21) Numéro de dépôt : **89905613.9**

(22) Date de dépôt : **31.05.89**

(86) Numéro de dépôt international :
**PCT/CH89/00102**

(87) Numéro de publication internationale :
**WO 90/00376 25.01.90 Gazette 90/03**

(54) **BALLON INTRAGASTRIQUE.**

(30) Priorité : **05.07.88 CH 2549/88**

(43) Date de publication de la demande :
**01.08.90 Bulletin 90/31**

(45) Mention de la délivrance du brevet :
**08.07.92 Bulletin 92/28**

(84) Etats contractants désignés :
**AT BE CH DE FR GB IT LI LU NL SE**

(56) Documents cités :
**EP-A- 0 103 481**
**EP-A- 0 241 038**
**WO-A-87/00034**

(73) Titulaire : **CANTENYS, José**
**CH-1501 Palézieux-Village (CH)**

(72) Inventeur : **CANTENYS, José**
**CH-1501 Palézieux-Village (CH)**

(74) Mandataire : **Rochat, Daniel Jean et al**
**Bovard SA Ingénieurs-Conseils ACP**
**Optingenstrasse 16**
**CH-3000 Bern 25 (CH)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 379 530 B1

Jouve, 18, rue Saint-Denis, 75001 PARIS

## Description

On connaît déjà des ballons intragastriques qui, placés dans l'estomac d'une personne obèse, diminuent la capacité de l'estomac, ce qui a pour effet une diminution de l'appétit et en conséquence, provoque une sensible perte de poids, cette perte de poids pouvant par ailleurs être augmentée si la personne suit, parallèlement au traitement par ballon intragastrique, un régime alimentaire. Toutefois, la mise en place desdits ballons intragastriques dans l'estomac de la personne n'est pas sans problème. En effet, cette mise en place nécessite une intervention ambulatoire, voire même une hospitalisation d'un jour, et, de plus, une anesthésie locale de la gorge est nécessaire lors de l'introduction du ballon; le ballon doit ensuite être gonflé à l'aide d'air comprimé, par exemple, de manière à obtenir le volume désiré. Le retrait du ballon après quelques semaines ou quelques mois, nécessite également une intervention ambulatoire; de plus, des contrôles réguliers, effectués par un médecin, sont indiqués. Ce traitement est donc non seulement astreignant, mais il est également onéreux.

Une autre proposition a été faite d'un ballon se gonflant sous l'action d'une réaction chimique provoquant le dégagement d'un gaz non toxique; les composants nécessaires à la réaction chimique sont mis en contact juste avant d'avaler le ballon, ou alors la réaction est amorcée par l'action des liquides situés dans l'estomac ou alors les composants sont isolés par du sucre ou matière semblable, se dissolvant sous l'action de l'eau (Voir EP-A-0 103 481).

Selon une autre proposition, le ballon est rempli à l'état dégonflé d'un liquide à bas point de fusion se transformant en gaz sous l'action de la température du corps humain.

Ces diverses propositions ont généralement l'inconvénient de ne pas dominer le moment du gonflement du ballon, ce gonflement intervenant alors trop tôt, avant que le ballon ne soit dans l'estomac, ceci pouvant amener toutes sortes d'ennuis à la personne en traitement. D'autre part, l'état de la technique précédent mentionne l'utilisation d'un ballon, voire de deux ou trois simultanément dans l'estomac; l'utilisation de ballons de dimensions relativement importantes peut conduire à de graves inconvénients, voire à des occlusions intestinales.

Le but de la présente invention est de réaliser un ballon intragastrique se gonflant sans moyens extérieurs à l'intérieur de l'estomac et qui, étant de faibles dimensions, peut facilement être éliminé par les voies naturelles lorsqu'il se sera dégonflé et qui, présent simultanément en nombre important, permet d'occuper un certain volume dans l'estomac d'une personne, et qui pallie les inconvénients ci-dessus.

A cet effet, le ballon est constitué par une enveloppe au moins approximativement étanche renfermant au moins une substance ou une composition qui permet de provoquer automatiquement et sans apport d'énergie externe, à l'intérieur de l'estomac de la personne, un gonflage de l'enveloppe, caractérisé en ce que ce gonflement provient d'une réaction chimique rendue possible par la fonte de l'enrobage de l'un des constituants.

D'autres caractéristiques de l'invention ressortiront de la description détaillée de l'objet de l'invention, en se référant au dessin annexé dans lequel

la fig.1 est une vue en élévation d'une forme d'exécution du ballon dans son état dégonflé,

la fig.2 est une vue en élévation d'une forme d'exécution du ballon durant la réaction chimique provoquant le gonflage de l'enveloppe pour former le ballon,

la fig.3 est une vue en perspective d'une forme d'exécution du ballon dans son état gonflé.

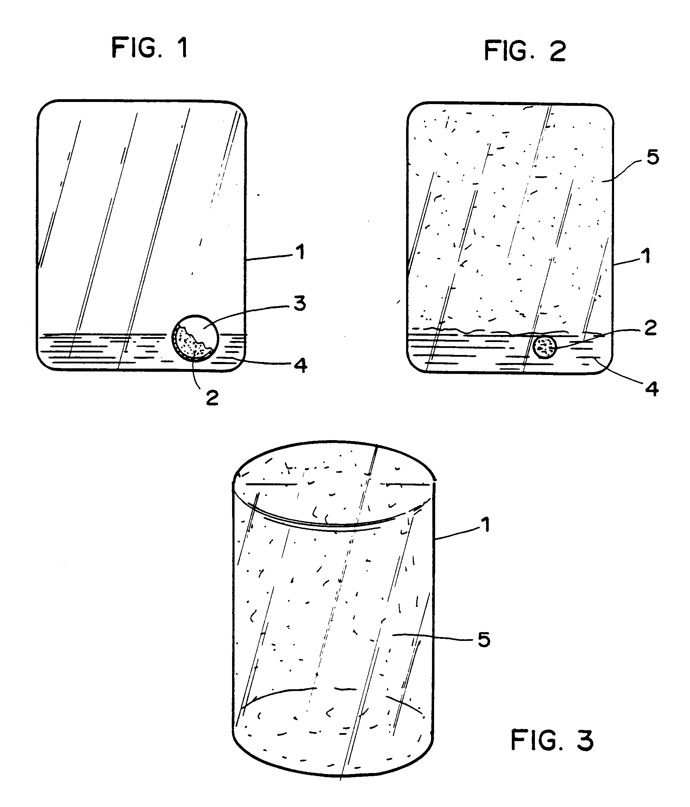

La fig.1 montre les principaux éléments d'une forme d'exécution de l'objet de l'invention, à savoir :
- l'enveloppe 1 hermétiquement close par soudure de sa périphérie, par exemple,
- une substance ou composition 2 réagissant chimiquement avec la substance 4 afin de former un gaz,
- l'enrobage 3 isolant la substance ou composition 2 de la substance 4 à une température inférieure à celle du corps humain.

Sur cette figure, la réaction chimique ne pouvant avoir lieu, le ballon est dégonflé et peut, vu ses faibles dimensions, être aisément avalé avec l'aide d'un peu d'eau, par exemple.

Dans une forme d'exécution de l'invention, l'enveloppe 1 peut être réalisée en matière synthétique souple telle que le polyéthylène, polypropylène, PVC, PVCD ,PET, Téflon ou tout autre type de matériau approprié. Le choix du matériau formant enveloppe, l'épaisseur du film de même que sa microporosité seront choisis de telle manière que la perméabilité de l'enveloppe permette au ballon de se dégonfler progressivement après quelques heures ou quelques jours, par exemple; on pourra ainsi moduler les périodes actives selon l'intensité des cures. Le ballon se dégonflera donc après un certain temps prédéterminé et sera évacué par les voies naturelles. La matière de l'enveloppe sera évidemment choisie afin qu'elle ne présente aucun danger d'intoxication pour la personne. Il en sera de même pour la substance ou composition 2, pour la substance 4, pour l'enrobage 3, ainsi que pour le gaz résultant de la réaction chimique. Dans une forme d'exécution de l'invention, la composition 2 pourrait être une pastille ou une poudre comprenant un acide solide et un carbonate ou bicarbonate non toxique, alors que la substance 4 serait de l'eau et l'enrobage 3 du chocolat, de la pâte ou du beurre de cacao. Il va de soi que d'autres formes d'exécutions, telles que, par exemple, de l'acide citrique et un bicarbonate alcalin enrobé de graisse végétale ou animale non toxique fondant à la température du corps humain et qui mis en présence d'eau, produiraient le même résultat.

La fig.2 représente le ballon, objet de la présente invention, lorsqu'il a été avalé et se trouve dans l'estomac. L'enrobage ayant fondu lentement sous l'action de la température régnant à l'intérieur de l'estomac, la composition 2 comprenant, par exemple, de l'acide citrique et le bicarbonate de sodium va engendrer du $CO_2$ 5 en réagissant en présence de l'eau 4 et va, de ce fait, commencer à gonfler l'enveloppe en vue de former le ballon.

La fig.3 représente le ballon, objet de la présente invention, entièrement gonflé par le $CO_2$. Le sel résultant de la réaction chimique, de même que l'enrobage fondu ne sont pas représentés.

La forme et les dimensions du ballon seront choisies en fonction de différents critères pratiques, à savoir: facilité d'avaler l'enveloppe, élimination des risques de lésions internes, volume désiré du ballon, etc. On pourra également prévoir un conditionnement, de telle sorte que l'enveloppe se présente, avant utilisation, enrobée dans une gélule facilement avalable, par exemple. Les dimensions réduites du ballon en permettent l'élimination avant qu'il soit complètement dégonflé; de ce fait le passage d'un ou plusieurs ballons à l'état semi-dégonflé dans le transit intestinal augmente la durée de ce transit, augmentant ainsi l'efficacité du traitement de réduction de poids, tout en évitant le risque d'une occlusion intestinale.

Le traitement consistera à avaler un nombre choisi de ballons, ceci en fonction de la diminution de la capacité de l'estomac désirée. Ensuite, lors d'un traitement continu, chaque ballon évacué par les voies naturelles sera remplacé par un nouveau ballon.

La diminution de la capacité de l'estomac provoquant une diminution de l'appétit de la personne, ce traitement entraînera une perte de poids sensible.

L'emploi d'un ballon se gonflant automatiquement sous l'effet de la température peut aussi être prévu pour d'autres usages que celui mentionné plus haut; en particulier comme témoin d'une température atteinte dans une enceinte ou par un élément quelconque, cette enceinte ou cet élément pouvant être de toute nature, aussi en dehors d'une application au corps humain ou animal, par exemple un élément mécanique quelconque, ou alors devant actionner un mécanisme quelconque en fonction d'une température de déclenchement. Le choix du composant isolant les constituants susceptibles de réagir chimiquement et fondant à une température donnée, devra alors être fait tenant compte de la température de déclenchement souhaitée; à cet effet, le dosage d'une charge d'huile dans une cire, par exemple, permet de choisir la température à laquelle cette cire fondra. De même, les autres caractéristiques du ballon, matière de l'enveloppe, dimensions et forme du ballon, constituants réagissant chimiquement et gaz gonflant seront choisis en fonction de l'application souhaitée.

## Revendications

1. Ballon intragastrique destiné à occuper un certain volume dans l'estomac d'une personne constitué par une enveloppe (1) au moins approximativement étanche renfermant une composition (2) de deux substances, soit un acide sous forme solide, par exemple de l'acide citrique, et d'un sel de carbonate, par exemple du bicarbonate de sodium, ainsi que de l'eau (4), réagissant chimiquement afin de former un gaz non toxique, par exemple du gaz carbonique, permettant de provoquer automatiquement et sans apport d'énergie ou de substance externe, à l'intérieur de l'estomac de la personne, un gonflage de l'enveloppe, caractérisé en ce que les deux substances sont enrobées d'une matière (3) les isolant de l'eau à une température inférieure à celle du corps humain, et fondant sous l'effet de la température du corps humain après que ledit ballon ait été avalé, ceci permettant la mise en contact des deux substances avec l'eau, provoquant de ce fait la réaction chimique.

2. Ballon intragastrique selon la revendication 1, caractérisé en ce que la matière enrobant les deux substances afin de les isoler de l'eau est une graisse végétale ou animale, en particulier du beurre de cacao ou de la pâte de cacao.

3. Ballon intragastrique selon la revendication 1, caractérisé en ce que l'enveloppe est en polyéthylène, polypropylène, PVC, PVCD, PET ou Téflon.

4. Ballon intragastrique selon la revendication 3, caractérisé en ce que la porosité de l'enveloppe est choisie afin de permettre le dégonflement du ballon après un temps prédéterminé.

5. Ballon intragastrique selon l'une des revendications précédentes, caractérisé en ce que ledit ballon est conditionné sous forme de gélule.

6. Ballon intragastrique selon l'une des revendications précédentes, caractérisé en ce que ledit ballon est de dimensions réduites à l'état gonflé, un nombre choisi de ballons permettant d'occuper le volume nécessaire de l'estomac.

7. Ballon autogonflable constitué par une enveloppe (1) au moins approximativement étanche renfermant une composition (2) de deux substances, soit un acide sous forme solide et un sel de carbonate ainsi que de l'eau (4), réagissant chimiquement afin de former un gaz permettant de provoquer automatiquement et sans apport d'énergie ou de substance externe un gonflage de l'enveloppe, caractérisé en ce que les deux substances sont enrobées d'une matière (3) les isolant de l'eau à une température inférieure à une température prédéterminée, et fondant sous l'effet de ladite température, ceci permettant la mise en contact des deux substances avec l'eau, provoquant de ce fait la réaction chimique, la composition de la matière enrobant les deux substances étant choisie afin de fondre et de déclencher la réaction chimique en fonction de ladite température prédéterminée.

**Patentansprüche**

1. Intragastraler Ballon, der dazu bestimmt ist, ein gewisses Volumen im Magen einer Person einzunehmen, umfassend eine Umhüllung (1), die mindestens angenähert dicht ist und eine Mischung (2) aus zwei Stoffen einschliesst, nämlich eine Säure in fester Form, beispielsweise Zitronensäure, und ein Carbonatsalz, beispielsweise Natriumbicarbonat ebenso wie Wasser (4), welche chemisch reagieren, um ein nicht toxisches Gas zu bilden, beispielsweise Kohlendioxid, welches automatisch und ohne Zuführung externer Energie oder einer externen Substanz im Innern des Magens der Person ein Aufblasen der Umhüllung ermöglicht, dadurch gekennzeichnet, dass die beiden Stoffe mit einem sie bei einer Temperatur, kleiner als diejenige des menschlichen Körpers, vom Wasser isolierenden Material (3) umhüllt sind, das unter Einwirkung der Temperatur des menschlichen Körpers schmilzt, nachdem der Ballon verschluckt wurde, was ermöglicht, dass die beiden Substanzen mit Wasser in Kontakt treten und dabei die chemische Reaktion ausgelöst wird.

2. Intragastraler Ballon nach Patentanspruch 1, dadurch gekennzeichnet, dass die Materie, welche die beiden Stoffe umhüllt um sie von Wasser zu isolieren, ein pflanzliches oder tierisches Fett ist, im speziellen Kakaobutter oder Kakaopaste.

3. Intragastraler Ballon nach Patentanspruch 1, dadurch gekennzeichnet, dass die Umhüllung aus Polyethylen, Polypropylen, PVC, PVCD, PET oder Teflon besteht.

4. Intragastraler Ballon nach Patentanspruch 2, dadurch gekennzeichnet, dass die Porosität der Umhüllung so gewählt wird, um ein Luftablassen des Ballons nach einer vorbestimmten Zeit zu ermöglichen.

5. Intragastraler Ballon nach einem der vorangehenden Patentansprüche, dadurch gekennzeichnet, dass der Ballon in Form einer Kapsel verpackt ist.

6. Intragastraler Ballon nach einem der vorangehenden Patentansprüche, dadurch gekennzeichnet, dass der Ballon im aufgeblasenen Zustand eine reduzierte Dimension aufweist, wobei eine ausgewählte Anzahl von Ballonen das notwendige Volumen des Magens belegen können.

7. Selbstaufblasbarer Ballon mit einer Umhüllung (1), welche mindestens angenähert dicht ist und eine Mischung (2) aus zwei Stoffen einschliesst, nämlich eine Säure in fester Form und ein Carbonatsalz ebenso wie Wasser (4), welche chemisch miteinander reagieren, um ein Gas zu bilden, welches ermöglicht, automatisch und ohne Zuführung externer Energie oder einer externen Substanz die Umhüllung aufzublasen, dadurch gekennzeichnet, dass die beiden Stoffe von einem Material (3) umhüllt sind, welches diese bei einer Temperatur, die kleiner ist als eine vorbestimmte Temperatur, von Wasser isoliert und welches unter Einwirkung der genannten Temperatur schmilzt, was ermöglicht, dass die beiden Stoffe mit Wasser in Kontakt treten und dabei eine chemische Reaktion auslösen, wobei die Zusammensetzung des Materials, welches die beiden Substanzen umhüllt, gewählt wird um zu schmelzen und die chemische Reaktion in Funktion der genannten vorbestimmten Temperatur auszulösen.

**Claims**

1. Intragastric balloon intended to occupy a certain volume in a person's stomach made up of an envelope (1) which is at least approximatively fluid-tight, enclosing a composition (2) of two substances, say an acid in solid form, for example citric acid, and a carbonate salt, for example sodium bicarbonate, as well as water, reacting chemically in order to form a non-toxic gas, for example carbon dioxide, permitting an inflation of the envelope within the person's stomach to be caused automatically and without recourse to any outside energy or substance, characterized in that the two substances are coated with a material (3) isolating them from the water at a temperature lower than that of the human body, and melting under the effect of human body temperature after the said balloon has been swallowed, this permitting the two substances to be put in contact with the water, thereby causing the chemical reaction.

2. Intragastric balloon according to claim 1, characterized in that the material coating the two substances in order to isolate them from the water is a vegetable or animal fat, particularly cocoa butter or cocoa paste.

3. Intragastric balloon according to claim 1, characterized in that the envelope is of polyethylene, polypropylene, PVC, PVCD, PET, or Teflon.

4. Intragastric balloon according to claim 3, characterized in that the porosity of the envelope is chosen in order to permit the deflation of the balloon after a predetermined time.

5. Intragastric balloon according to one of the preceding claims, characterized in that said balloon is packaged in the form of a capsule.

6. Intragastric balloon according to one of the preceding claims, characterized in that said balloon is of small size in the inflated state, a chosen number of balloons permitting the necessary volume of the stomach to be occupied.

7. Self-inflatable balloon made up of an envelope (1) which is at least approximately fluid-tight, enclosing a composition (2) of two substances, say an acid in solid form and a carbonate salt as well as water (4), reacting chemically in order to form a gas permitting an inflation of the envelope to be caused automatically and without recourse to any outside energy or substance, characterized in that the two substances are coated with a material (3) isolating them from the water at a temperature lower than a predetermined temperature, and melting under the effect of said temperature, this permitting the two substances to be put in contact with the water, thereby causing the chemical reaction, the composition of the material coating the two substances being chosen in order to melt and to start the chemical reaction as a function of said predetermined temperature.

# FIG. 1

# FIG. 2

# FIG. 3